# EUROPEAN PATENT SPECIFICATION

(11) **EP 2 542 223 B1**
(45) Date of publication and mention of the grant of the patent: **23.09.2015**
(21) Application number: 11750883.8
(22) Date of filing: 25.02.2011
(51) Int. Cl.: A61K 9/16, A61K 9/14, A61K 35/74, A61K 47/34, A61K 9/48, A61K 31/166, A61K 31/4468, A61K 31/5375

(54) **COMPLEX FORMULATION FOR ORAL ADMINISTRATION COMPRISING PROBIOTIC FORMULATION AND 5-HT4 RECEPTOR AGONIST AND METHOD FOR THE PREPARATION THEREOF**
KOMPLEXE FORMULIERUNG ZUR ORALEN VERABREICHUNG MIT EINER PROBIOTISCHEN FORMULIERUNG UND DEM REZEPTORAGONISTEN 5-HT4 SOWIE HERSTELLUNGSVERFAHREN DAFÜR
FORMULATION COMPLEXE POUR ADMINISTRATION ORALE COMPRENANT UNE FORMULATION PROBIOTIQUE ET UN AGONISTE DU RÉCEPTEUR 5-HT4, ET PROCÉDÉ DE PRÉPARATION ASSOCIÉ

(30) Priority: 04.03.2010 KR 20100019424
(43) Date of publication of application: 09.01.2013
(73) Proprietor: Hanmi Science Co., Ltd., Gyeonggi-do 445-813 (KR)
(72) Inventor: WOO, Jong Soo, Suwon-si Gyeonggi-do 440-710 (KR); PARK, Jae Hyun, Suwon-si Gyeonggi-do 443-470 (KR); KIM, Yong Il, Suwon-si Gyeonggi-do 442-150 (KR); KIM, Dong Ho, Suwon-si Gyeonggi-do 440-320 (KR); CHOI, Jun Young, Suwon-si Gyeonggi-do 440-710 (KR)
(74) Representative: Von Kreisler Selting Werner - Partnerschaft von Patentanwälten und Rechtsanwälten mbB
(86) International application number: PCT/KR2011/001359
(87) International publication number: WO 2011/108826

(56) References cited:
- WO-A1-97/25065
- JP-A- 11 139 978
- KR-A- 20040 077 244
- KR-A- 20050 083 711
- US-A1- 2003 007 962
- US-A1- 2004 120 931
- US-A1- 2005 266 069
- US-A1- 2008 213 320
- US-B2- 6 676 933

## Description

### FIELD OF THE INVENTION

The present invention relates to a complex formulation for oral administration comprising probiotic formulation and 5-hydroxytryptamine 4 (5-HT4) receptor agonist.

### BACKGROUND OF THE INVENTION

Functional dyspepsia is a clinical syndrome of the digestive system characterized by epigastric pain and discomfort, and feeling full accompanied by bloating, nausea or vomiting after eating, without evidence of an organopathy ascertainable by a general diagnostic device including an endoscope. The syndrome is caused by complex reasons throughout the digestive organs, and various medications are employed for its clinical treatment. However, functional dyspepsia has a wide variety of symptoms which are difficult to be objectively estimated and analyzed, and it is not easy to verify effectiveness of the medications employed for the treatment of functional dyspepsia.

Medications for selectively facilitating gastrointestinal motility are clinically very useful and a representative example is serotonin. Serotonin (5-hydroxytryptamine, 5-HT) is found in the stomach (90%) and central nervous system (4∼5%), and agonists of 5-HT3 and 5-HT4 receptors out of 14 serotonin receptor subtypes have been primarily developed for use in the gastrointestinal tract.

Mosapride (4-amino-5-chloro-2-ethoxy-N-((4-(4-fluorobenzyl)-2-morpholinyl)methyl)benzamide) is recognized as a selective serotonin 5-HT4 receptor agonist, which is a well-known contributor to a strong digestive movement or gastric emptying movement. Mosapride can enhance the gastric emptying movement by facilitating acetylcholine-release through 5-HT4 receptor of intestinalmyenteric plexus, improve epigastric symptoms of a chronic gastritis, and does not block a dopamine D2 receptor.

US2003/007962, for example, discloses a pharmaceutical composition comprising mosapride and pancreatin for the treatment of gastrointestinal discomfort, such as dyspepsia. The dosage forms are solid oral dosage forms that can comprise a core comprising pancreatin and a pharmaceutical excipient, an enteric coat surrounding the core and a drug-containing coat surrounding the enteric coat and comprising mosapride. The mosapride is released in the stomach, while the pancreatin downstream of the stomach.

Probiotics are live microorganisms which can maintain intestinal microbial balance in the gastrointestinal tract of the animal host including a human. The probiotics have beneficial effects in maintaining intestinal microbial balance, improving diarrhea caused by Rotavirus, alleviating antibiotic-related diarrhea, lactose intolerance and infant food allergic symptoms, and performing intestinal regulation. Lactic acid bacteria are the most common microbes used as probiotics, and they inhabit in the intestinal epithelial cells and excretes lactic acid, fatty acid, antibiotics, and H₂O₂ to inhibit the growth of pathogens. The intestinal lactic acid bacteria block the attachment of pathogens to the epithelium of the digestive tract to prevent pathogen-induced diseases, and produce the antibiotics to kill the diarrhea-inducing pathogens or inhibit their growth. Also, the lactic acid bacteria produce lactic acid and acetic acid to improve overall functions of the gastrointestinal tract, e.g., by increasing the digestive movement. Therefore, probiotic formulations prepared by using such probiotics may be effective in the treatment of the cryptogenic functional dyspepsia.

However, the 5-HT4 receptor agonist and probiotic formulation should be released at different sites in the intestinal tract in consideration of their effectiveness. Namely, the 5-HT4 receptor agonist should be quickly released in the stomach to speed up its effect, but the probiotics should be slowly released in the small or large intestine because the probiotics are apt to dissolve upon contact with the gastric fluids in the stomach.

The present inventors have achieved the present invention by developing a complex formulation for oral administration comprising a probiotic formulation and 5-HT4 receptor agonist, which can release the active ingredients quickly and stably in the gastrointestinal tract.

### SUMMARY OF THE INVENTION

Accordingly, it is an object of the present invention to provide a complex formulation for oral administration for the treatment of the functional dyspepsia or a gastrointestinal disorder, comprising a probiotic formulation and 5-HT4 receptor agonist.

It is another object of the present invention to provide a method for preparing the complex formulation for oral administration.

In accordance with one aspect of the present invention, there is provided a complex formulation for oral administration which comprises a capsule comprising a core containing probiotics and a pharmaceutically acceptable excipient; an enteric coating layer coated on the capsule; and a 5-HT4 receptor agonist layer containing a 5-HT4 receptor agonist, which is formed on the surface of the enteric coating layer.

In accordance with another aspect of the present invention, there is provided a method for preparing the complex formulation for oral administration, which comprises the steps of:
1) admixing probiotics and a pharmaceutically acceptable excipient to obtain a core;
2) filling the core to a capsule;
3) coating the capsule to form an enteric coating layer thereon; and
4) coating the enteric coating layer to form a 5-HT4 receptor agonist layer thereon.

### BRIEF DESCRIPTION OF THE DRAWINGS

The above and other objects and features of the present invention will become apparent from the following description of the invention, when taken in conjunction with the accompanying drawings, which respectively show:
Fig. 1: the dissolution profiles of the complex formulations of Examples 1 and 2, and Gasmotin; and
Fig. 2: the dissolution profiles of the complex formulation of Example 2 after aging under a high temperature condition.

### DETAILED DESCRIPTION OF THE INVENTION

In the present invention, there is provided a complex formulation for oral administration which comprises a capsule comprising a core containing a probiotic and a pharmaceutically acceptable excipient; an enteric coating layer coated on the capsule; and a 5-HT4 receptor agonist layer containing a 5-HT4 receptor agonist, which is formed on the surface of the enteric coating layer.

Each ingredient of the inventive complex formulation is described in detail as follows:

### <Capsule comprising a core containing probiotics>

The complex formulation of the present invention comprises a core containing probiotics as an active ingredient, and a pharmaceutically acceptable excipient.

In the present invention, the term "probiotics" refers to conventional microorganisms having a capability of producing lactic acid or acetic acid by using carbohydrates such as glucose and lactose, and the probiotics are classified into 12 families including *Lactobacillus, Lactococcus, Carnobacterium, Atopobium, Pediococcus, Tetragenococcus, Leuconostoc, Wisella, Oenococcus, Enterococcus, Strepcoccus* and *Vagococcus* (Kang kook-hee, Probiotic sitology, Publishing department in Sungkyunkwan university, 1996). The probiotics produce lactic acid through glucose fermentation, thereby producing a flavor, inhibiting pathogens, and degrading a protein to produce amino acids, peptides, flavouring materials and antibacterial materials which are essential in growing. Especially, the probiotics can enhance the overall functions of the gastrointestinal tract by improving intestinal microflora and intestinal regulation.

In the present invention, the probiotics are selected from the group consisting of *Bacillus cereus, Bacillus licheniformis, Bacillus subtilis, Bifidobacterium bifidum, Bifidobacterium infantis, Bifidobacterium longum, Enterococcus faecium, Enterococcus faecalis, Lactobacillus acidopilus, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lactobacillus helveticus, Lactobacillus rhamnosus, Lactobacillus reuteri, Lactobacillus sake, Lactococcus lactis, Streptococcus faecium, Streptococcus faecalis, Streptococcus agalactiae, Streptococcus mutans* and a mixture thereof. The probiotics may be contained in the core in an amount of at least one billion cells.

In the present invention, the core may be in the form of a granule, pellet, tablet and the like. The pharmaceutically acceptable excipient may be any of the conventional excipients employed in the preparation of a granule, pellet or tablet.

The core containing the probiotics is filled to a capsule. The capsule may be any conventional one used for the pharmaceutical products.

The capsule plays a role to separate the core containing the probiotics from an enteric coating layer, thereby blocking the pH variations of the enteric coating layer and preventing the probiotics from extinction due to the external exposure during the enteric coating process.

### <Enteric coating layer>

The enteric coating layer may comprise an enteric coating material selected from the group consisting of an enteric cellulose derivative selected from the group consisting of hydroxypropylmethylcellulose acetate succinate, hydroxypropylmethylcellulose phthalate, hydroxymethylethylcellulose phthalate, celluloseacetate phthalate, celluloseacetate malate, celluloseacetate succinate, cellulosebenzoate phthalate, cellulosepropionate phthalate, methylcellulose phthalate, carboxymethylethylcellulose, ethylhydroxyethylcellulose phthalate, carboxymethylethylcellulose and ethylhydroxyethylcellulose phthalate; an enteric acrylate copolymer selected from the group consisting of styrene acrylate copolymer, methacrylate ethylmethacrylate copolymer (e.g., Eudragit L100, Eudragit S, and Degussa), methacrylate ethylacrylate copolymer (e.g., Eudragit L100-55) and methylacrylate methacrylate octylacrylate copolymer; an enteric malate copolymer selected from the group consisting of vinylacetate malate anhydride copolymer, styrene malate anhydride copolymer, styrene malate monoester copolymer, vinylmethylether malate anhydride copolymer, ethylene malate anhydride copolymer, vinylbutylether malate anhydride copolymer, acrylonitrile methylacrylate malate anhydride copolymer and butylacrylate styrene malate anhydride copolymer; an enteric polyvinyl derivative selected from the group consisting of polyvinyl alcohol phthalate, polyvinylacetal phthalate, polyvinylbutyrate phthalate and polyvinylacetacetal phthalate; shellac; and a mixture thereof.

The enteric coating layer can be formed by dispersing or dissolving the enteric coating material in water or an organic solvent selected from the group consisting of ethanol, isopropanol, acetone and methylene chloride to prepare an enteric coating solution; and coating the enteric coating solution on the surface of the capsule.

The enteric coating layer may further comprise a pharmaceutically acceptable plasticizer selected from the group consisting of triacetin, myvacet^{R}, citrate ester, phthalate ester, dibutyl cebacate, cetyl alcohol, polyethylene glycol, glycerides and a mixture thereof, to achieve the enteric coating layer having an appropriate solubility and mechanical characteristics such as hardness. Also, the enteric coating layer may further comprise hydroxypropyl methylcellulose, hydroxypropyl cellulose or polyvinylpyrrolidone, in order to improve a viscosity of the polymers.

The enteric coating layer may be coated on the surface of the capsule in an amount of at least 5% by weight, preferably, 10 to 50% by weight, more preferably 15 to 30% by weight based on the total weight of the core.

The enteric coated formulation thus obtained can prevent the probiotics from acidic fluids in the stomach, and release the active ingredients effectively in the small intestine or the large intestine.

### <Separation layer>

The complex formulation of the present invention may further comprises a separation layer disposed between the enteric coating layer and a 5-HT4 receptor agonist layer. The separation layer may be formed by dissolving a component selected from the group consisting of a cellulose derivative such as hydroxypropylmethyl cellulose and hydroxypropyl cellulose, a glucose derivative, a polyvinyl derivative and a mixture thereof in a solvent such as distilled water; and coating the resulting mixture on the surface of the enteric coating layer.

Also, the separation layer may further comprise a plasticizer selected from the group consisting of triacetin, myvacet^{R}, citrate ester, phthalate ester, dibutyl cebacate, cetyl alcohol, polyethylene glycol, glyceride and a mixture thereof.

The separation layer may be coated on the surface of the enteric coated formulation in an amount of less than 15% by weight, preferably, 0.1 to 10% by weight, more preferably 1 to 5% by weight based on the total weight of the core.

### <5-HT4 receptor agonist layer>

In case the 5-HT4 receptor agonist is surrounded by the enteric coating layer, the 5-HT4 receptor agonist may be released in the small intestine or the large intestine, thereby inducing no quick movement of the gastrointestinal tract. Therefore, the 5-HT4 receptor agonist should not be coated with the enteric coating layer, but should be separately coated on the surface of the enteric coating layer.

In the present invention, the 5-HT4 receptor agonist layer can be formed by dispersing or dissolving the 5-HT4 receptor agonist such as cisapride, mosapride and itopride in an aqueous solution comprising a polymer selected from the group consisting of polyvinylpyrrolidone, polyvinyl alcohol, polyvinyl acetate, hydroxypropyl cellulose, methylcellulose, ethylcellulose, hydroxypropyl methylcellulose, sodium carboxymethyl cellulose and a mixture thereof, which is soluble or rapidly disintegrates in water; and laminating the mixture to the surface of the enteric coating layer. The 5-HT4 receptor agonist layer may further comprise additives such as a plasticizer, a colorant and an anti-static agent.

The amounts of the 5-HT4 receptor agonist layer may be limited by a desired dissolution profile, however it may be coated on the surface of the enteric coated formulation in an amount ranging from 0.1 to 50% by weight, preferably 1 to 30% by weight, more preferably, 5 to 15% by weight based on the total weight of the core.

In a preferred embodiment of the present invention, the 5-HT4 receptor agonist layer further comprises a basifying agent to improve aging-stability of the inventive complex formulation.

The basifying agent used in the present invention may be selected from the group consisting of meglumine, histidine, arginine, magnesium oxide, sodium phosphate, disodium hydrogenphosphate, potassium phosphate, dipotassium hydrogenphosphate, magnesium methasilicate aluminate, magnesium carbonate and sodium benzoate, and meglumine, histidine and arginine are preferred.

The basifying agent may be employed in an amount ranging from 1 to 500% by weight, preferably 10 to 250% by weight, more preferably 50 to 150% by weight based on the total weight of the 5-HT4 receptor agonist.

In the present invention, there is also provided a method for preparing the inventive complex formulation for oral administration, which comprises the steps of: 1) admixing probiotics and a pharmaceutically acceptable excipient to obtain a core; 2) filling the core to a capsule; 3) coating the capsule to form an enteric coating layer thereon; and 4) coating the enteric coating layer to form a 5-HT4 receptor agonist layer thereon.

The following Examples are intended to further illustrate the present invention without limiting its scope.

### Examples

### <Preparation of complex formulation for oral administration>

### Example 1

### Step 1: Preparation of a core

2,500 g of *Bacillus subtilis* and *Streptococcus faecium* strain culture (Institut Rosell Inc., Canada), 48 g of lactose, 26 g of talc, and 26 g of magnesium stearate were admixed together using a double con mixer at a rate of 10 rpm for 30 minutes. The mixture thus obtained was filled to capsules (about 10,000 capsules).

### Step 2: Preparation of an enteric coating layer

500 g of hydroxypropylmethyl cellulose phthalate, 30 g of Myvacet, 83 g of hydroxypropyl cellulose, and 30 g of concentrated glycerin were dissolved in 1,500 g of ethanol and 3,750 g of acetone. The resulting mixture was spray-coated on the capsules obtained in step 1 (about 3,250 g) to prepare about 10,000 enteric coated capsules.

### Step 3: Preparation of a 5-HT4 receptor agonist layer

52.9 g of mosapride citrate dehydrate, 260 g of hydroxypropylmethyl cellulose, 24 g of polyethyleneglycol, and 40 g of polyvinylpyrrolidone were dissolved in a mixture of 1,000 g of distilled water and 2,500 g of ethanol. The resulting mixture was spray-coated on the enteric coated capsule obtained in step 2 to obtain about 10,000 capsules of the inventive complex formulation.

### Example 2

### Step 1: Preparation of a core

2,500 g of *Bacillus subtilis* and *Streptococcus faecium* strain culture (Institut Rosell Inc., Canada), 48 g of lactose, 26 g of talc, and 26 g of magnesium stearate were admixed together using a double con mixer at a rate of 10 rpm for 30 minutes. The mixture thus obtained was filled to capsules (about 10,000 capsules).

### Step 2: Preparation of an enteric coating layer

500 g of hydroxypropylmethyl cellulose phthalate, 30 g of Myvacet, 83 g of hydroxypropyl cellulose, and 30 g of concentrated glycerin were dissolved in 1,500 g of ethanol and 3,750 g of acetone. The resulting mixture was spray-coated on the capsules obtained in step 1 (about 3,250 g) to prepare about 10,000 enteric coated capsules.

### Step 3: Preparation of a separation layer

650 g of hydroxypropylmethyl cellulose, and 6 g of polyethylene glycol were dissolved in 240 g of distilled water and 560 g of ethanol. The resulting mixture was spray-coated to the enteric coated capsules obtained in step 2.

### Step 4: Reparation of a 5-HT4 receptor agonist layer

52.9 g of mosapride citrate dehydrate, 260 g of hydroxypropylmethyl cellulose, 24 g of polyethyleneglycol, and 40 g of polyvinylpyrrolidone were dissolved in a mixture of 1,000 g of distilled water and 2,500 g of ethanol. The resulting mixture was spray-coated on the coated capsule obtained in step 3 to obtain about 10,000 capsules of the inventive complex formulation.

### Example 3

The procedure of Example 2 was repeated except for further employing 52.9 g of arginine in step 4 to prepare the inventive complex formulation.

### Example 4

The procedure of Example 2 was repeated except for further employing 52.9 g of histidine in step 4 to prepare the inventive complex formulation.

### Example 5

The procedure of Example 2 was repeated except for further employing 52.9 g of meglumine in step 4 to prepare the inventive complex formulation.

### Test Example 1: Dissolution profile test

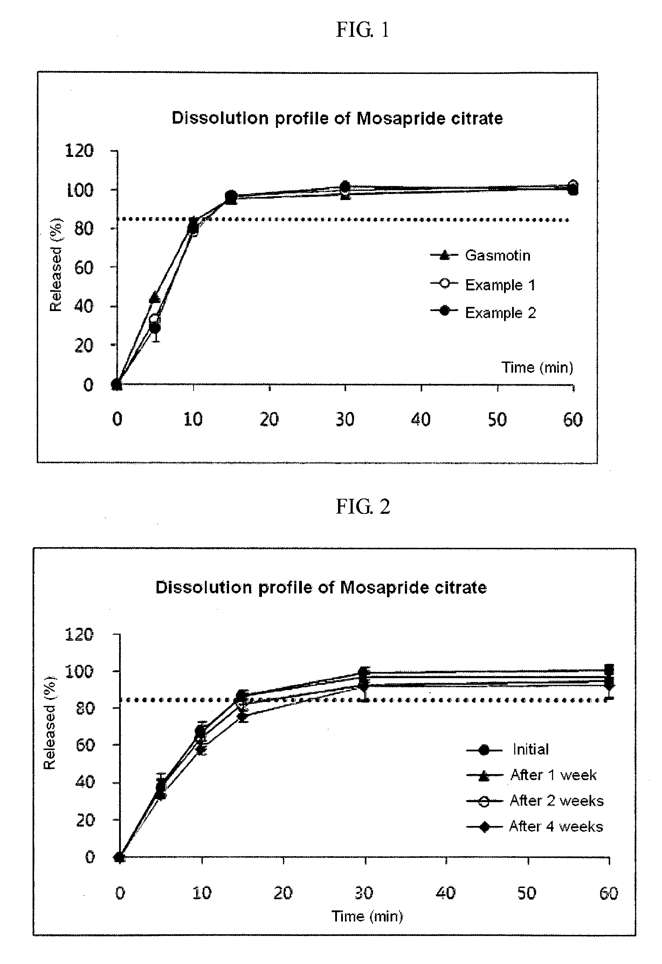

The complex formulations prepared in Examples 1 and 2 were compared with Gasmotin tablet (Daewoong Co. Ltd., mosapride citrate) by subjecting to a dissolution test using an artificial gastric fluid (pH 1.2), respectively, and the results are shown in Fig. 1. The dissolution test was performed by the paddle method using 900 ml of the artificial gastric fluid heated to 37 ± 0.5 °C at 50 rpm of a rotation speed. In case of the capsules, a sinker was used for aid.

As shown in Fig. 1, the release patterns of the complex formulations prepared in Examples 1 and 2 were similar to that of Gasmotin tablet, and thus it was concluded that the release rates of the active ingredients from the inventive complex formulation were not lowered by the spray-coating.

### Test Example 2: Stability under a high temperature condition

The complex formulation prepared in Example 2 was packed in a high density polyethylene bottle, and subjected to aging in a thermostat container under a high temperature condition of 60 ± 2°C. The samples after 1, 2 and 4 weeks were subjected to the dissolution test disclosed in Test Example 1, and the results are shown in Fig. 2.

As shown in Fig. 2, the inventive complex formulation is fully stable in the release rate after aging under the high temperature condition.

### Test Example 3: Stability

The complex formulations prepared in Examples 1 to 5 were packed in a high density polyethylene bottle, and each sample was subjected to aging in a thermostat container under a high temperature condition of 60 ± 5 °C. The amount of the 5-HT4 receptor agonist in each sample remained after 1, 2, 3 and 4 weeks relative to the initial amount were determined, and the results are shown in Table 1.

**<Table 1>**

| | Initial amount | Amount after 1 week | Amount after 2 weeks | Amount after 3 weeks | Amount after 4 weeks |
|---|---|---|---|---|---|
| Example 1 | 100% | 98.3% | 96.5% | 95.9% | 95.5% |
| Example 2 | 100% | 97.6% | 96.2% | 95.5% | 95.1% |
| Example 3 | 100% | 99.2% | 98.8% | 98.7% | 98.5% |
| Example 4 | 100% | 99.8% | 99.6% | 99.3% | 99.2% |
| Example 5 | 100% | 99.9% | 99.6% | 99.6% | 99.5% |

As shown in Table 1, the complex formulations of the present invention stably maintained the content of 5-HT4 receptor agonist, even when a basifying agent was comprised therein.

While the invention has been described with respect to the above specific embodiments, it should be recognized that various modifications and changes may be made to the invention by those skilled in the art which also fall within the scope of the invention as defined by the appended claims.

## Claims

1. A complex formulation for oral administration which comprises
a capsule comprising a core containing a probiotic and a pharmaceutically acceptable excipient;
an enteric coating layer coated on the capsule; and
a 5-HT4 receptor agonist layer containing a 5-HT4 receptor agonist, which is formed on the surface of the enteric coating layer.

2. The complex formulation for oral administration of claim 1, wherein the probiotic is selected from the group consisting of *Bacillus cereus, Bacillus licheniformis, Bacillus subtilis, Bifidobacterium bifidum, Bifidobacterium infantis, Bifidobacterium longum, Enterococcus faecium, Enterococcus faecalis, Lactobacillus acidopilus, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lactobacillus helveticus, Lactobacillus rhamnosus, Lactobacillus reuteri, Lactobacillus sake, Lactococcus lactis, Streptococcus faecium, Streptococcus faecalis, Streptococcus agalactiae, Streptococcus mutans,* and a mixture thereof.

3. The complex formulation for oral administration of claim 1, wherein the core contains the probiotic in an amount of at least one billion cells.

4. The complex formulation for oral administration of claim 1, wherein the enteric coating layer comprises an enteric coating material selected from the group consisting of an enteric cellulose derivative selected from the group consisting of hydroxypropylmethylcellulose acetate succinate, hydroxypropylmethylcellulose phthalate, hydroxymethylethylcellulose phthalate, celluloseacetate phthalate, celluloseacetate malate, celluloseacetate succinate, cellulosebenzoate phthalate, cellulosepropionate phthalate, methylcellulose phthalate, carboxymethylethylcellulose, ethylhydroxyethylcellulose phthalate, carboxymethylethylcellulose and ethylhydroxyethylcellulose phthalate; an enteric acrylate copolymer selected from the group consisting of styrene acrylate copolymer, methacrylate ethylmethacrylate copolymer, methacrylate ethylacrylate copolymer and methylacrylate methacrylate octylacrylate copolymer; an enteric malate copolymer selected from the group consisting of vinylacetate malate anhydride copolymer, styrene malate anhydride copolymer, styrene malate monoester copolymer, vinylmethylether malate anhydride copolymer, ethylene malate anhydride copolymer, vinylbutylether malate anhydride copolymer, acrylonitrile methylacrylate malate anhydride copolymer and butylacrylate styrene malate anhydride copolymer; an enteric polyvinyl derivative selected from the group consisting of polyvinylalcohol phthalate, polyvinylacetal phthalate, polyvinylbutyrate phthalate and polyvinylacetacetal phthalate; shellac; and a mixture thereof.

5. The complex formulation for oral administration of claim 4, wherein the enteric coating layer further comprises a plasticizer selected from the group consisting of triacetin, myvacet, citrate ester, phthalate ester, dibutyl cebacate, cetyl alcohol, polyethylene glycol, glycerides, and a mixture thereof.

6. The complex formulation for oral administration of claim 1, wherein the enteric coating layer is in an amount ranging from 10 to 50% by weight based on the total weight of the core.

7. The complex formulation for oral administration of claim 1, wherein the 5-HT4 receptor agonist is cisapride, mosapride or itopride.

8. The complex formulation for oral administration of claim 1, wherein the 5-HT4 receptor agonist layer further comprises polyvinylpyrrolidone, polyvinyl alcohol, polyvinyl acetate, hydroxypropyl cellulose, methylcellulose, ethylcellulose, hydroxypropyl methylcellulose, sodium carboxymethylcellulose, or a mixture thereof.

9. The complex formulation for oral administration of claim 1, wherein the 5-HT4 receptor agonist layer is in an amount ranging from 0.1 to 50% by weight based on the total weight of the core.

10. The complex formulation for oral administration of claim 1, which further comprises a separation layer disposed between the enteric coating layer and the 5-HT4 receptor agonist layer.

11. The complex formulation for oral administration of claim 10, wherein the separation layer comprises a cellulose derivative, a glucose derivative, a polyvinyl derivative, or a mixture thereof.

12. The complex formulation for oral administration of claim 10, wherein the separation layer is present in an amount ranging from 0.1 to 10% by weight based on the total weight of the core.

13. The complex formulation for oral administration of claim 1, wherein the 5-HT4 receptor agonist layer further comprises a basifying agent.

14. The complex formulation for oral administration of claim 13, wherein the basifying agent is selected from the group consisting of meglumine, histidine, arginine, magnesium oxide, sodium phosphate, disodium hydrogenphosphate, potassium phosphate, dipotassium hydrogenphosphate, magnesium methasilicate aluminate, magnesium carbonate, and sodium benzoate, and wherein the basifying agent is present in an amount ranging from 1 to 500% by weight based on the total weight of the 5-HT4 receptor agonist.

15. A method for preparing the complex formulation for oral administration of claim 1, which comprises the steps of:
1) admixing probiotics and a pharmaceutically acceptable excipient to obtain a core;
2) filling the core to a capsule;
3) coating the capsule to form an enteric coating layer thereon; and
4) coating the enteric coating layer to form a 5-HT4 receptor agonist layer thereon.

## Patentansprüche

1. Komplexe Zubereitung für die orale Verabreichung, umfassend:
eine Kapsel, die einen Kern umfasst, der ein Probiotikum und einen pharmazeutisch annehmbaren Arzneimittelhilfsstoff enthält;
eine magensaftresistente Beschichtung, die auf die Kapsel aufgetragen ist; und
eine 5-HT4-Rezeptor-Agonisten-Schicht, die einen 5-HT4-Rezeptor-Agonisten enthält, der auf der Oberfläche der magensaftresistenten Beschichtung gebildet ist.

2. Komplexe Zubereitung für die orale Verabreichung gemäß Anspruch 1, wobei das Probiotikum aus der Gruppe ausgewählt ist, die aus *Bacillus cereus, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lacrobacillus helveticus, Lactobacillus rhamnosus, Lactobacillus reuteri, Lactobacillus sake, Lactococcus lactis, Streptococcus faecium, Streptococcus faecalis, Streptococcus agalactiae, Streptococcus mutans* und einem Gemisch davon besteht.

3. Komplexe Zubereitung für die orale Verabreichung gemäß Anspruch 1, wobei der Kern das Probiotikum in einer Menge von wenigstens einer Milliarde Zellen enthält.

4. Komplexe Zubereitung für die orale Verabreichung gemäß Anspruch 1, wobei die magensaftresistente Beschichtung ein magensaftresistentes Beschichtungsmaterial umfasst, das aus der Gruppe ausgewählt ist, die aus Folgendem besteht: einem magensaftresistenten Cellulosederivat, das aus der Gruppe ausgewählt ist, die aus Hydroxypropylmethylcelluloseacetatsuccinat, Hydroxypropylmethylcellulosephthalat, Hydroxymethylethylcellulosephthalat, Celluloseacetatphthalat, Celluloseacetatmalat, Celluloseacetatsuccinat, Cellulosebenzoatphthalat, Cellulosepropionatphthalat, Methylcellulosephthalat, Carboxymethylethylcellulose, Ethylhydroxyethylcellulosephthalat, Carboxymethylethylcellulose und Ethylhydroxyethylcellulosephthalat besteht; einem magensaftresistenten Acrylat-Copolymer, das aus der Gruppe ausgewählt ist, die aus Styrol-Acrylat-Copolymer, Methacrylat-Ethylmethacrylat-Copolymer, Methacrylat-Ethylacrylat-Copolymer und Methylacrylat-Methacrylat-Octylacrylat-Copolymer besteht; einem magensaftresistenten Malat-Copolymer, das aus der Gruppe ausgewählt ist, die aus Vinylacetat-Äpfelsäureanhydrid-Copolymer, Styrol-Äpfelsäureanhydrid-Copolymer, Styrol-Äpfelsäuremonoester-Copolymer, Vinylmethylether-Äpfelsäureanhydrid-Copolymer, Ethylen-Äpfelsäureanhydrid-Copolymer, Vinylbutylether-Äpfelsäureanhydrid-Copolymer, Acrylnitril-Methylacrylat-Äpfelsäureanhydrid-Copolymer und Butylacrylat-Styrol-Äpfelsäureanhydrid-Copolymer besteht; einem magensaftresistenten Polyvinylderivat, das aus der Gruppe ausgewählt ist, die aus Polyvinylalkoholphthalat, Polyvinylacetalphthalat, Polyvinylbutyratphthalat und Polyvinylacetacetalphthalat besteht; Schellack; und einem Gemisch davon.

5. Komplexe Zubereitung für die orale Verabreichung gemäß Anspruch 4, wobei die magensaftresistente Beschichtung zusätzlich einen Weichmacher umfasst, der aus der Gruppe ausgewählt ist, die aus Triacetin, Myvacet, Zitronensäureester, Phthalsäureester, Dibutylsebacat, Cetylalkohol, Polyethylenglycol, Glyceriden und einem Gemisch davon besteht.

6. Komplexe Zubereitung für die orale Verabreichung gemäß Anspruch 1, wobei die magensaftresistente Beschichtung in einer Menge vorliegt, die im Bereich von 10 bis 50 Gew.-% liegt, bezogen auf das Gesamtgewicht des Kerns.

7. Komplexe Zubereitung für die orale Verabreichung gemäß Anspruch 1, wobei es sich bei dem 5-HT4-Rezeptor-Agonisten um Cisaprid, Mosaprid oder Itoprid handelt.

8. Komplexe Zubereitung für die orale Verabreichung gemäß Anspruch 1, wobei die 5-HT4-Rezeptor-Agonisten-Schicht weiterhin Polyvinylpyrrolidon, Polyvinylalkohol, Polyvinylacetat, Hydroxypropylcellulose, Methylcellulose, Ethylcellulose, Hydroxypropylmethylcellulose, Natriumcarboxymethylcellulose oder ein Gemisch davon umfasst.

9. Komplexe Zubereitung für die orale Verabreichung gemäß Anspruch 1, wobei die 5-HT4-Rezeptor-Agonisten-Schicht in einer Menge vorliegt, die im Bereich von 0,1 bis 50 Gew.-% liegt, bezogen auf das Gesamtgewicht des Kerns.

10. Komplexe Zubereitung für die orale Verabreichung gemäß Anspruch 1, die weiterhin eine Trennschicht umfasst, die sich zwischen der magensaftresistenten Beschichtung und der 5-HT4-Rezeptor-Agonisten-Schicht befindet.

11. Komplexe Zubereitung für die orale Verabreichung gemäß Anspruch 10, wobei die Trennschicht ein Cellulosederivat, ein Glucosederivat, ein Polyvinylderivat oder ein Gemisch davon umfasst.

12. Komplexe Zubereitung für die orale Verabreichung gemäß Anspruch 10, wobei die Trennschicht in einer Menge vorliegt, die im Bereich von 0,1 bis 10 Gew.-% liegt, bezogen auf das Gesamtgewicht des Kerns.

13. Komplexe Zubereitung für die orale Verabreichung gemäß Anspruch 1, wobei die 5-HT4-Rezeptor-Agonisten-Schicht weiterhin ein Alkalisierungsmittel umfasst.

14. Komplexe Zubereitung für die orale Verabreichung gemäß Anspruch 13, wobei das Alkalisierungsmittel aus der Gruppe ausgewählt ist, die aus Meglumin, Histidin, Arginin, Magnesiumoxid, Natriumphosphat, Dinatriumhydrogenphosphat, Kaliumphosphat, Dikaliumhydrogenphosphat, Magnesiummetasilicataluminat, Magnesiumcarbonat und Natriumbenzoat besteht und wobei das Alkalisierungsmittel in einer Menge vorliegt, die im Bereich von 1 bis 500 Gew.-% liegt, bezogen auf das Gesamtgewicht des 5-HT4-Rezeptor-Agonisten.

15. Verfahren zur Herstellung der komplexen Zubereitung für die orale Verabreichung gemäß Anspruch 1, das die folgenden Schritte umfasst:
1) Mischen von Probiotika und eines pharmazeutisch annehmbaren Arzneimittelhilfsstoffs, wobei man einen Kern erhält;
2) Füllen des Kerns in eine Kapsel;
3) Beschichten der Kapsel unter Bildung einer magensaftresistenten Beschichtung auf derselben; und
4) Beschichten der magensaftresistenten Beschichtung unter Bildung einer 5-HT4-Rezeptor-Agohisten-Schicht auf derselben.

## Revendications

1. Formulation complexe pour administration par voie orale, comprenant
une capsule comprenant un noyau contenant un agent probiotique et un excipient pharmaceutiquement acceptable ;
une couche d'enrobage entérique revêtant la capsule ; et
une couche d'agoniste du récepteur 5-HT4 contenant un agoniste du récepteur 5-HT4, formée sur la surface de la couche d'enrobage entérique.

2. Formulation complexe pour administration par voie orale selon la revendication 1, dans laquelle ledit agent probiotique est choisi dans le groupe constitant en *Bacillus cereus, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus bulgaricus, Lactobacillus casei, Lactobacillus curvatus, Lactobacillus delbrukii, Lactobacillus johnsonii, Lactobacillus farciminus, Lactobacillus gasseri, Lactobacillus helveticus, Lactobacillus rhamnosus, Lactobacillus reuteri, Lactobacillus sake, Lactococcus lactis, Streptococcus faecium, Streptococcus faecalis, Streptococcus agalactiae, Streptococcus mutans* et un mélange de ceux-ci.

3. Formulation complexe pour administration par voie orale selon la revendication 1, dans laquelle ledit noyau contient ledit agent probiotique en une quantité d'au moins un milliard de cellules.

4. Formulation complexe pour administration par voie orale selon la revendication 1, dans laquelle ladite couche d'enrobage entérique comprend un matériau d'enrobage entérique choisi dans le groupe constitant en un dérivé de cellulose entérique choisi dans le groupe constitant en l'acétatesuccinate d'hydroxypropylméthylcellulose, le phtalate d'hydroxypropylméthylcellulose, le phtalate d'hydroxyméthyléthylcellulose, l'acétate-phtalate de cellulose, l'acétate-malate de cellulose, l'acétatesuccinate de cellulose, le benzoate-phtalate de cellulose, le propionatephtalate de cellulose, le phtalate de méthylcellulose, la carboxyméthyléthylcellulose, le phtalate d'éthylhydroxyéthylcellulose, la carboxyméthyléthylcellulose et le phtalate d'éthylhydroxyéthylcellulose, un copolymère d'acrylate entérique choisi dans le groupe constitant en copolymère styrène-acrylate, copolymère méthacrylate-méthacrylate d'éthyle, copolymère méthacrylate-acrylate d'éthyle et copolymère acrylate de méthyle-méthacrylate-acrylate d'octyle; un copolymère malate entérique choisi dans le groupe constitant en copolymère acétate de vinyle-anhydride malique, copolymère styrène-anhydride malique, copolymère styrènemonoester d'acide malique, copolymère éther méthylvinylique-anhydride malique, copolymère éthylène-anhydride malique, copolymère éther butylvinylique-anhydride malique, copolymère acrylonitrile-acrylate de méthyle-anhydridemalique et copolymère acrylate de butyle-styrène-anhydride malique ; un dérivé de polyvinyle entérique choisi dans le groupe constitant en phtalate d'alcool polyvinylique, phtalate d'acétal polyvinylique, phtalate de polybutyrate de vinyle et phtalate d'acétacétal polyvinylique ; la gomme-laque; et un mélange de ceux-ci.

5. Formulation complexe pour administration par voie orale selon la revendication 4, dans laquelle ladite couche d'enrobage entérique comprend en outre un plastifiant choisi dans le groupe constitant en triacétine, Myvacet, ester d'acide citrique, ester d'acide phtalique, sébacate de dibutyle, alcool cétylique, polyéthylène glycol, glycérides et un mélange de ceux-ci.

6. Formulation complexe pour administration par voie orale selon la revendication 1, dans laquelle ladite couche d'enrobage entérique est présente en une quantité comprise entre 10 et 50 % en poids par rapport au poids total du noyau.

7. Formulation complexe pour administration par voie orale selon la revendication 1, dans laquelle ledit agoniste du récepteur 5-HT4 est le cisapride, le mosapride ou l'itopride.

8. Formulation complexe pour administration par voie orale selon la revendication 1, dans laquelle ladite couche d'agoniste du récepteur 5-HT4 comprend en outre de la polyvinylpyrrolidone, de l'alcool polyvinylique, de l'acétate de polyvinyle, de la hydroxypropylcellulose, de la méthylcellulose, de l'éthylcellulose, de la hydroxypropylméthylcellulose, de la carboxyméthylcellulose de sodium ou un mélange de ceux-ci.

9. Formulation complexe pour administration par voie orale selon la revendication 1, dans laquelle ladite couche d'agoniste du récepteur 5-HT4 est présente en une quantité comprise entre 0,1 et 50 % en poids par rapport au poids total du noyau.

10. Formulation complexe pour administration par voie orale selon la revendication 1, comprenant en outre une couche de séparation disposée entre ladite couche d'enrobage entérique et ladite couche d'agoniste du récepteur 5-HT4.

11. Formulation complexe pour administration par voie orale selon la revendication 10, dans laquelle ladite couche de séparation comprend un dérivé de cellulose, un dérivé de glucose, un dérivé de polyvinyle ou un mélange de ceux-ci.

12. Formulation complexe pour administration par voie orale selon la revendication 10, dans laquelle ladite couche de séparation est présente en une quantité comprise entre 0,1 et 10 % en poids par rapport au poids total du noyau.

13. Formulation complexe pour administration par voie orale selon la revendication 1, dans laquelle ladite couche d'agoniste du récepteur 5-HT4 comprend en outre un agent d'alcalinisation.

14. Formulation complexe pour administration par voie orale selon la revendication 13, dans laquelle ledit agent d'alcalinisation est choisi dans le groupe constitant en méglumine, histidine, arginine, oxyde de magnésium, phosphate de sodium, hydrogénophosphate de disodium, phosphate de potassium, hydrogénophosphate de dipotassium, métasilicate-aluminate de magnésium, carbonate de magnésium, et benzoate de sodium, et dans laquelle ledit agent d'alcalinisation est présent en une quantité comprise entre 1 et 500 % en poids par rapport au poids total de l'agoniste du récepteur 5-HT4.

15. Procédé pour préparer la formulation complexe pour administration par voie orale selon la revendication 1, comprenant les étapes consistant à :
1) mélanger des agents probiotiques et un excipient pharmaceutiquement acceptable pour obtenir un noyau ;
2) remplir le noyau pour former une capsule ;
3) revêtir la capsule pour former une couche d'enrobage entérique sur celle-ci ; et
4) revêtir la couche d'enrobage entérique pour former une couche d'agoniste du récepteur 5-HT4 sur celle-ci.
